# EUROPEAN PATENT APPLICATION

(11) **EP 1 623 973 A1**
(43) Date of publication of application: **08.02.2006**
(21) Application number: 04729305.5
(22) Date of filing: 23.04.2004
(51) Int. Cl.: C07C 251/24, C07D 213/28, C07D 215/40, C07D 239/26, C07D 295/12, C07D 307/58, C07D 317/48, C07D 319/18, C07D 333/20, C07D 333/58, C07D 471/04, C07F 15/00

(54) **PLATINUM COMPLEX**

(30) Priority: 30.04.2003 JP 2003124840
(71) Applicant: Takasago International Corporation, Tokyo-to 144-8721 (JP)
(72) Inventor: NAKAYAMA, Yuji; c/o Central Research Lab, Hiratsuka-shi Kanagawa 2540073 (JP); IWATA, Takeshi; c/o Central Research Lab Takasago, Kanagawa 2540073 (JP); MATSUSHIMA, Yoshimasa; c/o Central Research Lab, Hiratsuka-shi Kanagawa 2540073 (JP); HORI, Yoji; c/o Central Research Lab of Takasago, Kanagawa 2540073 (JP)
(74) Representative: Neidl-Stippler, Cornelia
(86) International application number: PCT/JP2004/005940
(87) International publication number: WO 2004/096755

(57) **Abstract**

A platinum complex represented by the following formula (1) or (2): 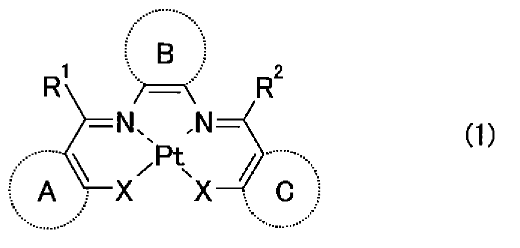 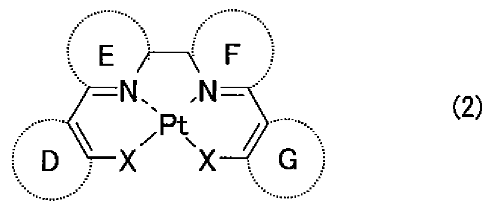
wherein, ring A, ring B, ring C, ring D and ring G each independently represent an aromatic ring optionally having substituent(s) or an aromatic heterocyclic ring optionally having substituent(s); X represents an oxygen atom or a sulfur atom; R¹ and R² each independently represent a hydrogen atom or a substituent; the ring E and the ring F each independently represent a nitrogen-containing aromatic heterocyclic ring, and more R¹ and the ring B, and also R² and the ring B each may combine together to form a fused ring and that the ring E and the ring F may combine together to form a fused ring. The platinum complex is useful for a luminescent device of an organic EL device or the like, and can provide a device being extremely good in thermal stability, emission characteristics and power efficiency.

## Description

### TECHNICAL FIELD

The present invention relates to new platinum complexes useful, for example, as light-emitting materials that can be used favorably in a display device, a display device, a back light unit, an electrophotographic machine, an illumination light source, a record light source, a light-exposure source, a read light source, signs and marks, a signboard, interior goods, or the like.

### BACKGROUND ART

Various research and development of display devices have been eagerly conducted recently, and among them, organic electroluminescent devices (hereinafter, referred to as "organic EL devices") capable of emitting high-brightness light at low voltage are attracting attention as a promising next-generation display device. The organic EL device is faster in response than the liquid crystal device traditionally used as a display device. In addition, because of self-emitting the organic EL device does not need back light units as in the liquid crystal display device, and allows production of an extremely thinner flat panel display. The organic EL device, that is a light-emitting device utilizing an electroluminescence (EL) phenomenon, is similar in principle to LEDs, and is characteristic in that it uses organic compounds as its light-emitting materials. As an example of the organic EL device using such an organic compound as the light-emitting material, there were reported organic EL devices utilizing multilayered thin films formed by evaporative deposition. The light-emitting device, which has deposited layers of an tris(8-hydroxyquinolinato-O,N)aluminum (Alq₃) as electron transporting material and a layer comprising a hole transporting material (e.g., aromatic amine compound), is significantly improved in emission characteristics, compared with conventional single-layer devices.

It is necessary, for development of a high-performance multi-colored display, to improve the properties and efficiency of the light-emitting devices respectively in the three primary colors of light, red, green and blue. Use of a phosphorescent material for the light-emitting layer was also proposed as a means of improving the properties of the organic EL device. Phosphorescence is a light emission phenomenon from a triplet excited state, and known to show quantum efficiency higher than that of the fluorescence, that is a light emission phenomenon from a singlet excited state. It would be possible to achieve a high power efficiency by using organic compounds having such properties as light-emitting materials.

As such an organic EL device using the phosphorescent substance, a device using an orthometalated iridium complex, tris(2-phenylpyridinato-N,C²)iridium (III), in the light-emitting layer was already reported. The report discloses that the iridium complex is a green phosphorescent substance higher in color purity that has an extremely favorable external quantum efficiency of 9%. However, there are still no red or blue phosphorescent substances superior both in color purity and power efficiency. For example, a device using a platinum complex, (2,3,7,8,12,13,17,18-octaethyl-21H,23H-porphinato-N,N,N,N)platinum (II) [Pt(OEP)] in a light-emitting layer was reported as the organic EL device using a red phosphorescent material (e. g. , M. A. Baldo et al., Nature, Vol. 395, 151-154 (1998)) . The platinum complex is a red phosphorescent substance higher in color purity, but the external quantum efficiency thereof is still approximately 4% as determined by the measuring method described in the literature, demanding further improvement in power efficiency. More recently, a device using bis[2-(2'-benzothienyl)pyridinato-N,C^{3'}]iridium (III) acetylacetonate [Ir(btp)₂(acac)] in the light-emitting layer has been reported. This platinum complex has a relatively favorable external quantum efficiency of approximately 7%, but is far lower in color purity than Pt(OEP).

On the other hand, as a platinum complex, [[2,2'-[1,2-phenylenebis(nitrilomethylidyne)]bis[phenolate]]-N,N',O,O']platinum (II)is known and phenomena of UV light absorption and fluorescence thereof are reported (e.g., M. E. Ivanova et al., Zhur. Fiz. Khim., Vol.65, 2957-2964 (1991)).

However, there was no report on application of the compound to light-emitting devices, synthesis of the derivatives obtained by substitutive modification of the ring in the ligand by substituent (s) or structural modification of the ring itself, or the like. In addition, even though the compound shows such phenomena of UV light absorption and fluorescence, it is not clear whether the compound or the derivative thereof shows such a phosphorescence phenomenon when used in organic EL devices, or whether it shows properties better than those of the devices currently used.

As described above, various studies for making next-generation display devices fit for practical use are being conducted. Among these, organic EL devices using a phosphorescent material are spotlighted particularly from the viewpoint of improvement in properties. However, the research has been started only recently, and there are still many problems pending, for example, of improvement in the light-emission characteristics, power efficiency, and color purity of the device and optimization of its structure. To solve the problems above, it is desired to develop new phosphorescent materials and methods of supplying the material efficiently.

An object of the present invention, which was made considering the problems above, is to provide platinum complexes useful, for example, as materials for light-emitting device and extremely favorable in heat stability, light-emission characteristics and power efficiency.

### DISCLOSURE OF THE INVENTION

As a result of eager study and examination to solve the problems above, the present inventors have found that platinum complexes having a particular structure showed superior heat stability, light-emission characteristics and power efficiency. After further studies based on the finding for production of devices using the platinum complex, they found that the platinum complex was extremely favorable as a phosphorescent material for a light-emitting device, and completed the present invention.

Accordingly, the present invention relates to a platinum complex represented by general formula (1): 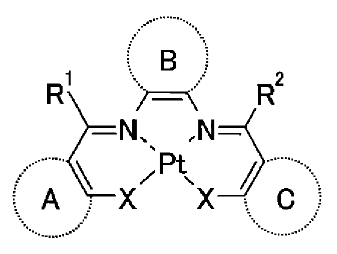 (wherein, rings A, B and C each independently represent an aromatic ring optionally having substituent (s) or an aromatic heterocyclic ring optionally having substituent (s) ; X represents an oxygen atom or a sulfur atom; R¹ and R² each independently represent a hydrogen atom or a substituent; and more, R¹ and the ring B, and also R² and the ring B each may combine together to form a fused ring.), with the proviso that excluding the following platinum complex:

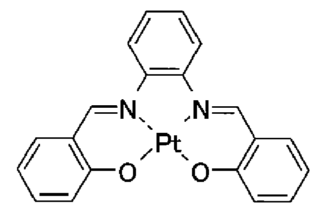

In addition, the present invention relates to a platinum complex represented by general formula (2): 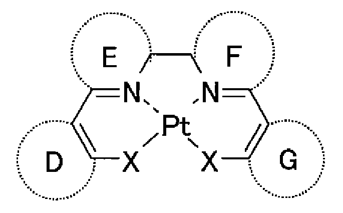
wherein, rings D and G each independently represent an aromatic ring optionally having substituent (s) or an aromatic heterocyclic ring optionally having substituent (s) ; X represents an oxygen atom or a sulfur atom; rings E and F each independently represent a nitrogen-containing aromatic heterocyclic ring optionally having substituent (s) ; and more, rings E and F may combine together to form a fused ring.

### BRIEF DESCRIPTION OF THE DRAWING

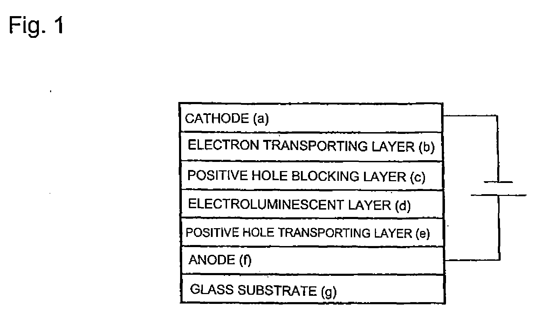

Fig. 1 is a schematic sectional view illustrating the layer structure of an organic EL device.

### DETAILED DESCRIPTION OF THE INVENTION

Hereinafter, the platinum complexes of the present invention represented by the general formulae (1) and (2) will be described in more detail.

In the general formulae (1) and (2), rings A, B, C, D, and G each independently represent an aromatic ring optionally having substituent (s) or an aromatic heterocyclic ring optionally having substituent(s). The aromatic ring includes, for example, monocyclic, polycyclic and fused aromatic rings having 6 to 14 carbons, and specific examples thereof include benzene, naphthalene, anthracene, and phenanthrene rings, and the like. The substituted aromatic rings include rings in which at least one hydrogen atom of the aromatic rings above is substituted with a substituent.

The aromatic heterocyclic ring includes an aromatic heterocyclic ring of, for example, a five- to eight-membered, preferably five- or six-membered, monocyclic, polycyclic or fused ring having 2 to 15 carbon atoms and having one or more, preferably one to three, heteroatoms such as a nitrogen atom, an oxygen atom, and a sulfur atom, and specific examples thereof include furan, thiophene, pyridine, pyrimidine, pyrazine, pyridazine, pyrazoline, imidazole, oxazole, thiazole, benzofuran, benzothiophene, quinoline, isoquinoline, quinoxaline, phthalazine, quinazoline, naphthyridine, cinnoline, benzimidazole, benzoxazole, and benzothiazole rings, and the like. The substituted aromatic heterocyclic ring includes a substituted aromatic heterocyclic ring in which at least one hydrogen atom of the aromatic heterocyclic ring above is substituted with a substituent.

Examples of the substituent above include a hydrocarbyl group, an aliphatic heterocyclic group, an aromatic heterocyclic group, a hydroxyl group, an alkoxy group, an alkylenedioxy group, an aryloxy group, an aralkyloxy group, a heteroaryloxy group, an acyloxy group, an acyl group, a carboxyl group, an alkoxycarbonyl group, an aryloxycarbonyl group, an aralkyloxycarbonyl group, a mercapto group, an alkylthio group, an arylthio group, an aralkylthio group, a heteroarylthio group, a sulfino group, a sulfinyl group, a sulfo group, a sulfonyl group, an amino group, a substituted amino group, a carbamoyl group, a substituted carbamoyl group, a sulfamoyl group, a substituted sulfamoyl group, an ureido group, a substituted ureido group, a phosphoric amide group, a silyl group, a hydrazino group, a cyano group, a nitro group, a hydroxamic acid group, halogen atoms, and the like.

More specifically, typical examples of the hydrocarbyl group include an alkyl group, an alkenyl group, an alkynyl group, an aryl group, an aralkyl group, and the like. Among these groups, the alkyl group may be a straight-chain, branched, or cyclic alkyl group having, for example, 1 to 15 carbon atoms, preferably 1 to 10 carbon atoms, and more preferably 1 to 6 carbons; and specific examples thereof include methyl, ethyl, n-propyl, 2-propyl, n-butyl, 2-butyl, isobutyl, tert-butyl, n-pentyl, 2-pentyl, tert-pentyl, 2-methylbutyl, 3-methylbutyl, 2,2-dimethylpropyl, n-hexyl, 2-hexyl, 3-hexyl, tert-hexyl, 2-methylpentyl, 3-methylpentyl, 4-methylpentyl, 2-methylpentan-3-yl, cyclopropyl, cyclobutyl, cyclopentyl, and cyclohexyl groups, and the like. The alkenyl group includes a straight-chain or branched alkenyl group, for example, having 2 to 15 carbon atoms, preferably having 2 to 10 carbon atoms, and more preferably having 2 to 6 carbons; and specific examples thereof include ethenyl, propenyl, 1-butenyl, pentenyl, and hexenyl groups, and the like. The alkynyl group includes a straight-chain or branched alkynyl group having, for example, 2 to 15 carbon atoms, preferably 2 to 10 carbon atoms, and more preferably 2 to 6 carbons; and specific examples thereof include ethynyl, 1-propynyl, 2-propynyl, 1-butynyl, 3-butynyl, pentynyl, andhexynylgroups, and the like. The aryl group includes an aryl group having, for example, 6 to 14 carbons; and specific examples thereof include phenyl, naphthyl, anthryl, phenanthrenyl, and biphenyl groups, and the like. The aralkyl group includes an aralkyl group in which at least one hydrogen atom of the alkyl group above is substituted with the aryl group. Preferable aralkyl groups are aralkyl groups having, for example, 7 to 13 carbons, and specific examples thereof include benzyl, 2-phenylethyl, 1-phenylpropyl, and 3-naphthylpropyl groups, and the like.

The aliphatic heterocyclic group includes, for example, a five- to eight-membered, preferably five- or six-membered, monocyclic,polycyclic orfused ring aliphatic heterocyclic group, having 2 to 14 carbon atoms and one or more, preferably one to three, heteroatoms such as a nitrogen atom, an oxygen atom, and a sulfur atom. Typical examples of the aliphatic heterocyclic group include pyrrolidyl-2-one, piperidino, piperadinyl, morpholino, tetrahydrofuryl, tetrahydropyranyl, and tetrahydrothienyl groups, and the like.

Further, the aromatic heterocyclic group is, for example, a five- to eight-membered, preferably five- or six-membered, monocyclic, polycyclic or fused ring heteroaryl group having 2 to 15 carbon atoms and one or more, preferably one to three, heteroatoms such as a nitrogen atom, an oxygen atom, and a sulfur atom; and specific examples thereof include furyl, thienyl, pyridyl, pyrimidyl, pyrazyl, pyridazyl, pyrazolyl, imidazolyl, oxazolyl, thiazolyl, benzofuryl, benzothienyl, quinolyl, isoquinolyl, quinoxalyl, phthalazyl, quinazolyl, naphthyridyl, cinnolyl, benzimidazolyl, benzoxazolyl, and benzothiazolyl groups, and the like.

The alkoxy group includes a straight-chain, branched, or cyclic alkoxy group having, for example, 1 to 6 carbons; and specific examples thereof include methoxy, ethoxy, n-propoxy, 2-propoxy, n-butoxy, 2-butoxy, isobutoxy, tert-butoxy, n-pentyloxy, 2-methylbutoxy, 3-methylbutoxy, 2,2-dimethylpropyloxy, n-hexyloxy, 2-methylpentyloxy, 3-methylpentyloxy, 4-methylpentyloxy, 5-methylpentyloxy, and cyclohexyloxy groups, and the like.

The alkylenedioxy group includes an alkylenedioxy group having, for example, 1 to 3 carbons; and specific examples thereof include methylenedioxy, ethylenedioxy, and propylenedioxy groups, and the like.

The aryloxy group includes an aryloxy group having, for example, 6 to 14 carbons, and specific examples thereof include phenyloxy, naphthyloxy, and anthryloxy groups, and the like.

The aralkyloxy group includes an aralkyloxy group having, for example, 7 to 12 carbons, and specific examples thereof include benzyloxy, 2-phenylethoxy, 1-phenylpropoxy, 2-phenylpropoxy, 3-phenylpropoxy, 1-phenylbutoxy, 2-phenylbutoxy, 3-phenylbutoxy, 4-phenylbutoxy, 1-phenylpentyloxy, 2-phenylpentyloxy, 3-phenylpentyloxy, 4-phenylpentyloxy, 5-phenylpentyloxy, 1-phenylhexyloxy, 2-phenylhexyloxy, 3-phenylhexyloxy, 4-phenylhexyloxy, 5-phenylhexyloxy, and 6-phenylhexyloxy groups, and the like.

The heteroaryloxy group includes a heteroaryloxy group having, for example, 2 to 14 carbons and having one or more, preferably one to three, heteroatom(s) such as a nitrogen atom, an oxygen atom, and a sulfur atom as a foreign atom; and specific examples thereof include 2-pyridyloxy, 2-pyrazyloxy, 2-pyrimidyloxy, and 2-quinolyloxy groups and the like.

The acyloxy group includes a carboxylic acid-derived acyloxy group having, for example, 2 to 18 carbons, and specific examples thereof include acetoxy, propionyloxy, butyryloxy, pivaloyloxy, pentanoyloxy, haxanoyloxy, lauroyloxy, stearoyloxy, and benzoyloxy groups, and the like.

The acyl group includes a straight-chain or branched acyl group having, for example, 1 to 18 carbon atoms derived from a fatty or aromatic carboxylic acid, and specific examples thereof include formyl, acetyl, propionyl, butyryl, pivaloyl, pentanoyl, haxanoyl, lauroyl, stearoyl, and benzoyl groups, and the like.

The alkoxycarbonyl group includes a straight-chain, branched, or cyclic alkoxycarbonyl group having, for example, 2 to 19 carbon atoms, and specific examples thereof include methoxycarbonyl, ethoxycarbonyl, n-propoxycarbonyl, 2-propoxycarbonyl, n-butoxycarbonyl, tert-butoxycarbonyl, pentyloxycarbonyl, hexyloxycarbonyl, 2-ethylhexyloxycarbonyl, lauryloxycarbonyl, stearyloxycarbonyl, and cyclohexyloxycarbonyl groups, and the like.

The aryloxycarbonyl group includes an aryloxycarbonyl group having, for example, 7 to 20 carbon atoms, and specific examples thereof include phenoxycarbonyl and naphthyloxycarbonyl groups, and the like. The aralkyloxycarbonyl group includes an aralkyloxycarbonyl group having, for example, 8 to 15 carbon atoms; and specific examples thereof include benzyloxycarbonyl, phenylethoxycarbonyl, and 9-fluorenylmethyloxycarbonyl groups, and the like.

The alkylthio group includes a straight-chain, branched, or cyclic alkylthio group having, for example, 1 to 6 carbon atoms, and specific examples thereof include methylthio, ethylthio, n-propylthio, 2-propylthio, n-butylthio, 2-butylthio, isobutylthio, tert-butylthio, pentylthio, hexylthio, and cyclohexylthio groups, and the like. The arylthio group includes an arylthio group having, for example, 6 to 14 carbons, such as a phenylthio or naphthylthio group. The aralkylthio group includes an axalkylthio group having, for example, 7 to 12 carbons, such as a benzylthio or 2-phenethylthio group. The heteroarylthio group is, for example, a heteroarylthio group having 2 to 14 carbons and one or more, preferably one to three, heteroatoms such as nitrogen, oxygen, and sulfur, and specific examples thereof include 4-pyridylthio, 2-benzimidazolylthio, 2-benzoxazolylthio, and 2-benzothiazolylthio groups, and the like.

The sulfinyl group includes a substituted sulfinyl group represented by, for example, R-SO- (R represents one of the alkyl, aryl, and aralkyl groups above, or the like.). Typical examples of the sulfinyl group include methanesulfinyl and benzenesulfinyl groups, and the like. The sulfonyl group includes a substituted sulfonyl group represented by, for example, R-SO₂- (R represents one of the alkyl, aryl, and aralkyl groups above or the like.). Typical examples of the sulfonyl group include methanesulfonyl and p-toluenesulfonyl groups, and the like.

The substituted amino group includes, for example, a substituted amino group in which one or two hydrogen atoms of an amino group are substituted with substituent (s) such as the alkyl and aryl groups described above or an amino group-protecting group. The protecting group is not particularly limited if it is used as an amino protecting group, and examples thereof include the amino protecting groups described in PROTECTIVE GROUPS IN ORGANIC SYNTHESIS, Second Edition, John Wiley & Sons, Inc. Typical examples of the amino protecting group include an aralkyl group, an acyl group, an alkoxycarbonyl group, an aryloxycarbonyl group, an aralkyloxycarbonyl group, and a sulfonyl group, and the like.

Typical examples of the amino group substituted with alkyl group(s), i.e., an alkyl group-substituted amino group, include mono- or di-alkylamino group such as N-methylamino, N,N-dimethylamino, N,N-diethylamino, N,N-diisopropylamino, and N-cyclohexylamino groups, and the like. Typical examples of the amino group substituted with aryl group(s), i.e., an aryl group-substituted amino group, include mono- ordi-arylaminogroup such as N-phenylamino, N,N-diphenylamino, N-naphthylamino, and N-naphthyl-N-phenylamino group, and the like. Typical examples of the amino group substituted with aralkyl group(s), i.e., an aralkyl group-substituted amino group include mono- or di-aralkylamino group such as N-benzylamino and N, N-dibenzylamino groups, and the like.

Typical examples of the amino group substituted with an acyl group, i.e., an acylamino group, include formylamino, acetylamino, propionylamino, pivaloylamino, pentanoylamino, haxanoylamino, and benzoylamino groups, and the like. Typical examples of the amino group substituted with an alkoxycarbonyl group, i.e., analkoxycarbonylamino group, include methoxycarbonylamino, ethoxycarbonylamino, n-propoxycarbonylamino, n-butoxycarbonylamino, tert-butoxycarbonylamino, pentyloxycarbonylamino, and hexyloxycarbonylamino groups, and the like. Typical examples of the amino group substituted with an aryloxycarbonyl group, i.e., an aryloxycarbonylamino group, include phenoxycarbonylamino and naphthyloxycarbonylamino groups, and the like. Typical examples of the amino group substituted with an aralkyloxycarbonyl group, i.e., an aralkyloxycarbonylamino group, include a benzyloxycarbonylamino group and the like . Typical examples of the amino group substituted with a sulfonyl group, i.e., a sulfonylamino group, include methanesulfonylamino and p-toluenesulfonylamino groups, and the like.

Examples of the substituted carbamoyl group include a substituted carbamoyl group in which one or two hydrogen atoms of an amino group in a carbamoyl group are substituted with substituent (s) such as the alkyl, aryl, and aralkyl groups above; and specific examples thereof include N-methylcarbamoyl, N,N-diethylcarbamoyl, and N-phenylcarbamoyl groups, and the like. Examples of the substituted sulfamoyl group include a substituted sulfamoyl group in which one or two hydrogen atoms of an amino group in a sulfamoyl group are subsituted with substituent (s) such as the alkyl, aryl, and aralkyl groups above; and specific examples thereof include N-methylsulfamoyl, N,N-dimethylsulfamoyl, and N-phenylsulfamoyl groups, and the like. Examples of the substituted ureido group include a substituted ureido group in which at least one hydrogen atom bound to a nitrogen atom of an ureido group is substituted with a substituent such as the alkyl group, aryl group, and aralkyl group described above, and specific examples thereof include N-methylureido and N-phenylureido groups, and the like.

Examples of the phosphoric amide group include a substituted phosphoric amide group in which one or more hydrogen atoms of a phosphate group in the phosphoric amide group are substituted with substituent(s) such as the alkyl, aryl, and aralkyl groups, and specific examples thereof include diethylphosphoric amide and phenylphosphoric amide groups, and the like. Examples of the silyl group include, for example, tri-substituted silyl groups having a silicon atom of which three hydrogen atoms are substituted with substituent (s) such as the alkyl, aryl, and aralkyl groups above; and specific examples thereof include trimethylsilyl, tert-butyldimethylsilyl, tert-butyldiphenylsilyl, and triphenylsilyl groups, and the like. Examples of the halogen atom include a fluorine atom, a chlorine atom, a bromine atom, and an iodine atom.

Among these substituents, the hydrocarbyl, aliphatic heterocyclic, aromatic heterocyclic, alkoxy, alkylenedioxy, aryloxy, aralkyloxy, heteroaryloxy, acyloxy, acyl, alkoxycarbonyl, aryloxycarbonyl, aralkyloxycarbonyl, alkylthio, arylthio, aralkylthio, heteroarylthio, sulfinyl, sulfonyl, substituted amino, substituted carbamoyl, substituted sulfamoyl, substituted ureido, phosphoric amide or silyl group may be substituted additionally with substituent(s) selected from the group of the substituents above.

In the general formulae (1) and (2), X represents an oxygen atom or a sulfur atom, and particularly preferable example of X is the oxygen atom.

In the general formula (1), R¹ and R² each independently represent a hydrogen atom or a substituent. The substituent includes, for example, a hydrocarbyl group, an aliphatic heterocyclic group, an aromatic heterocyclic group, and the like. Examples of the hydrocarbyl group, aliphatic heterocyclic group and aromatic heterocyclic group include those described in detail in the description of the substituents for the rings A, B, C, D and G. These substituents may additionally be substituted with substituent(s) selected from the group consisting of the substituents described in detail in the description for the rings A, B, C, D and G. In addition, R¹ and ring B, and R² and ring B each may combine together to form a fused ring. Typical examples of the fused ring include a quinoline ring, a dihydroquinoline ring, a quinazoline ring, a quinoxaline ring, a naphthyridine ring, and the like.

In the general formula (2), rings E and F each independently represent a nitrogen-containing aromatic heterocyclic ring optionally having substituent(s). The nitrogen-containing aromatic heterocyclic ring optionally having substituent(s) include, for example, a nitrogen-containing aromatic heterocyclic ring and a substituted nitrogen-containing aromatic heterocyclic ring. The nitrogen-containing aromatic heterocyclic ring is, for example, an aromatic heterocyclic ring having 2 to 15 carbon atoms and having one or more nitrogen atoms capable of coordinating to platinum. The nitrogen-containing aromatic heterocyclic ring may additionally have one to three heteroatom(s) such as a nitrogen atom, an oxygen atom, and a sulfur atom. In addition, two atoms next to the nitrogen atom coordinating to the platinum are preferably carbon atoms. Further, the nitrogen-containing aromatic heterocyclic ring is afive- to eight-membered,preferably five- or six-membered, monocyclic, polycyclic or fused nitrogen-containing aromatic heterocyclic ring. Typical examples of the nitrogen-containing aromatic heterocyclic ring include pyridine ring, pyrimidine ring, pyrazine ring, imidazole ring, oxazole ring, thiazole ring, isoquinoline ring, quinazoline ring, naphthyridine ring, and the like. The substituted nitrogen-containing aromatic heterocyclic ring is, for example, a nitrogen-containing aromatic heterocyclic ring of which one or more hydrogen atoms are substituted with substituent(s). Examples of the substituent include those described in detail in the description of the substituents for the rings A, B, C, D and G. In addition, the rings E and F may combine together to form a fused ring. Typical examples of the fused ring include 1, 10-phenanthroline ring and 4, 5-diazafluoren-9-one ring, and the like.

Typical examples of the platinum complex represented by the general formula (1) (excluding [[2,2'-[1,2-phenylene-bis(nitrilomethylidyne)]bis[phenolate]]-N,N',O,O']platinum (II)) and the platinum complex represented by the general formula (2) of the invention include the compounds represented by the following Formulae (1-1) to (1-76) and (2-1) to (2-9), and the like.

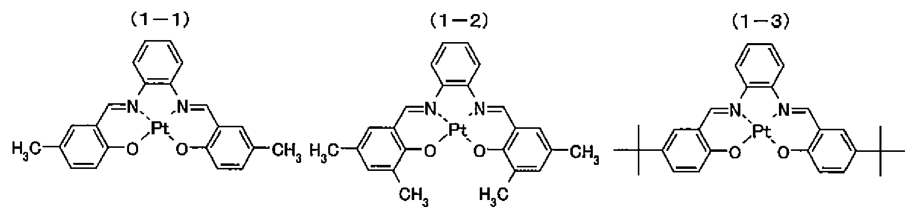

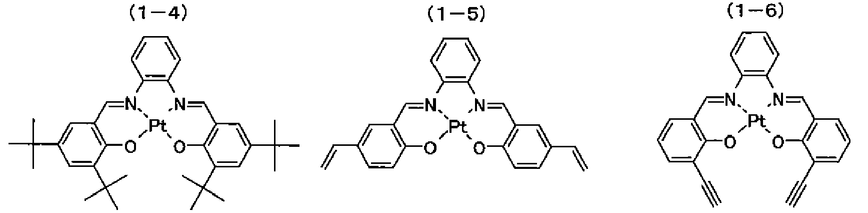

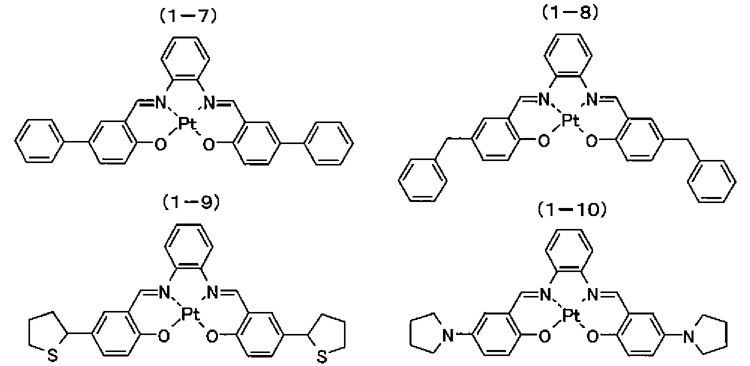

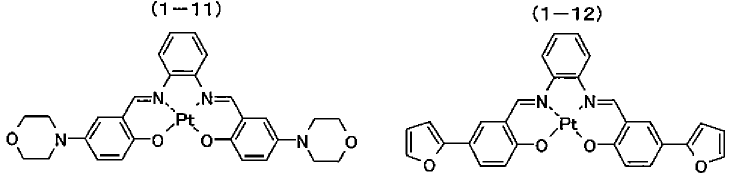

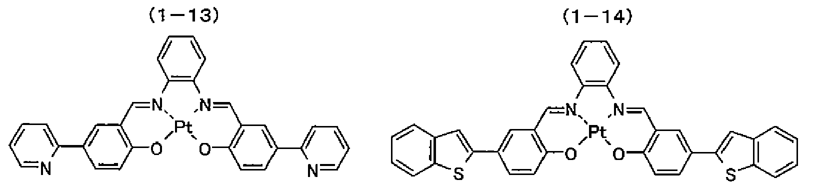

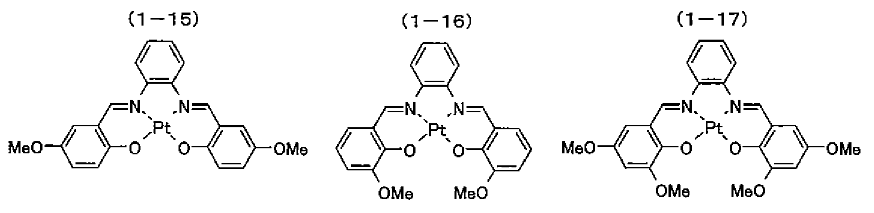

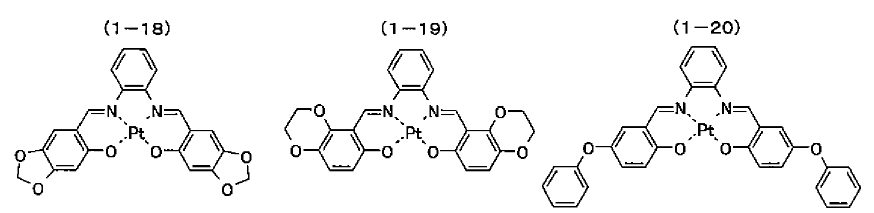

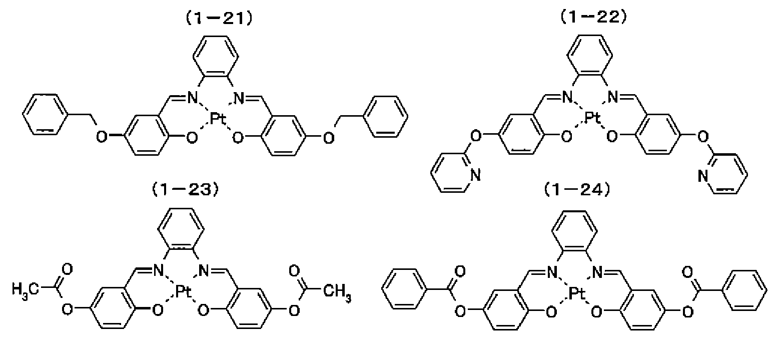

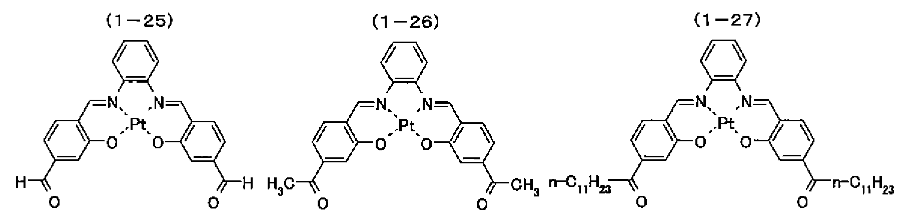

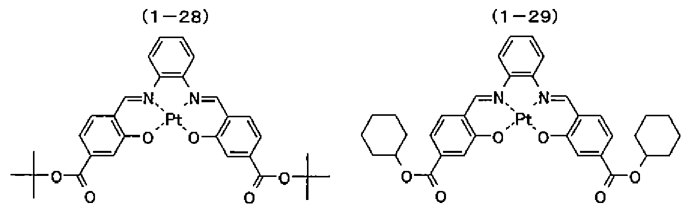

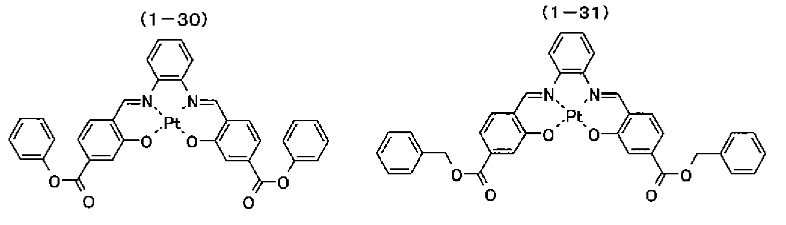

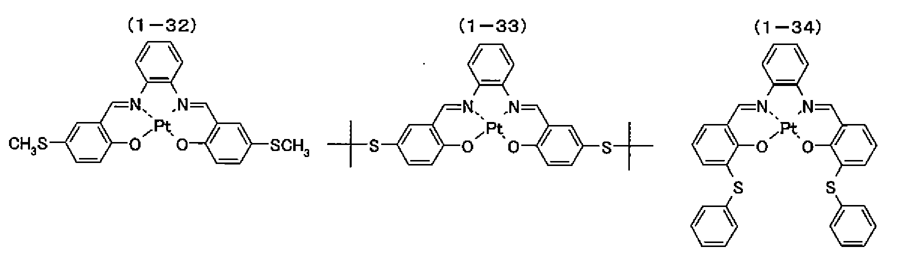

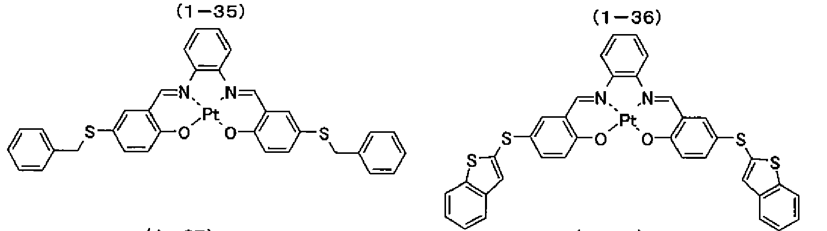

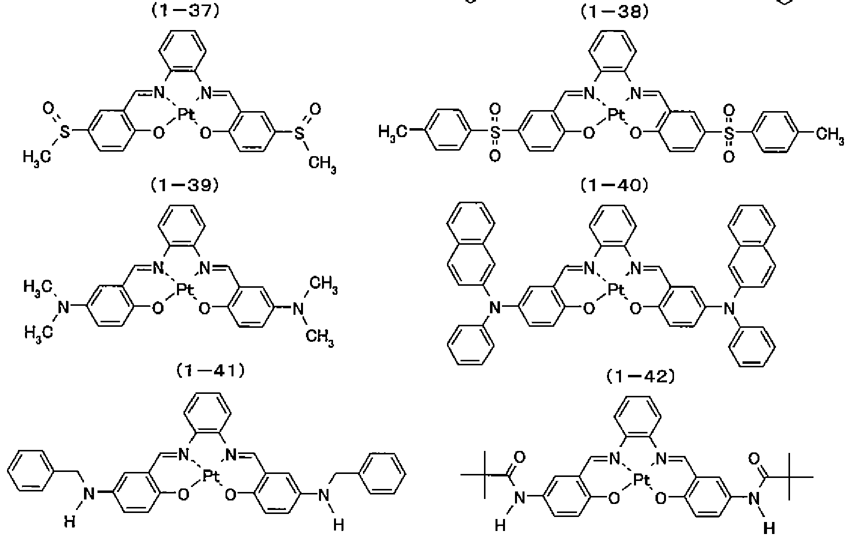

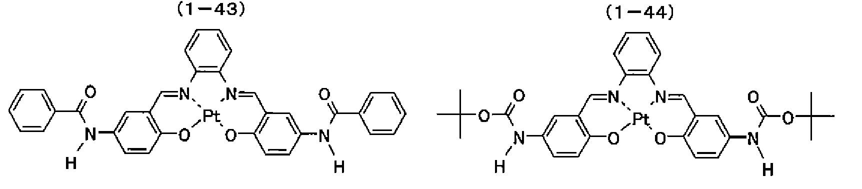

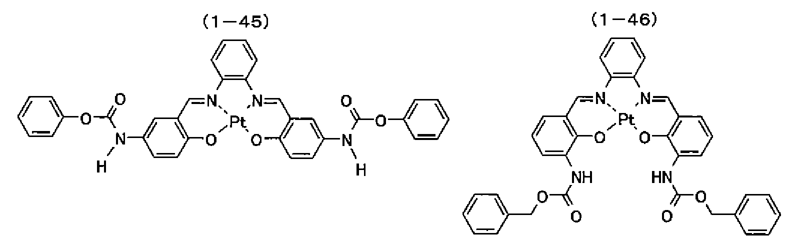

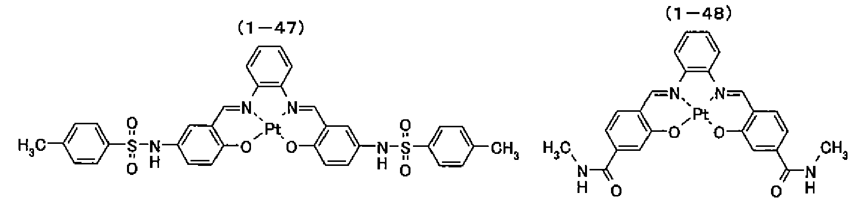

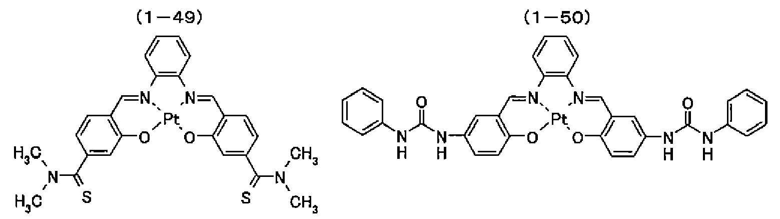

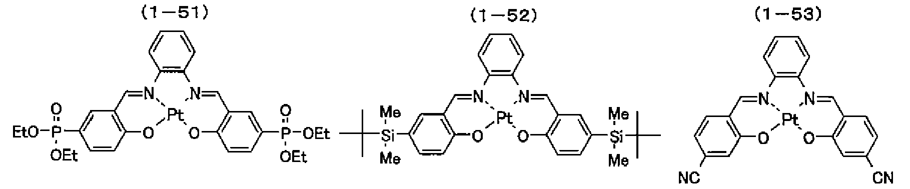

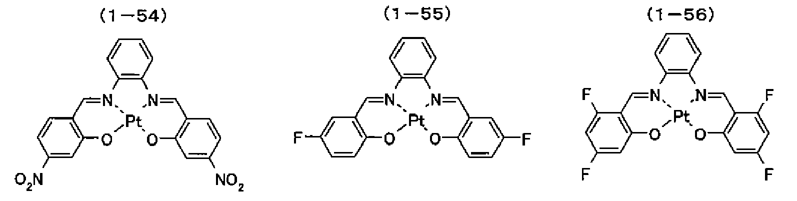

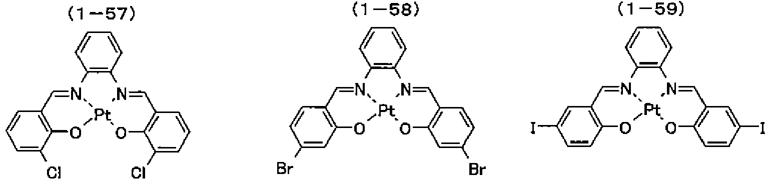

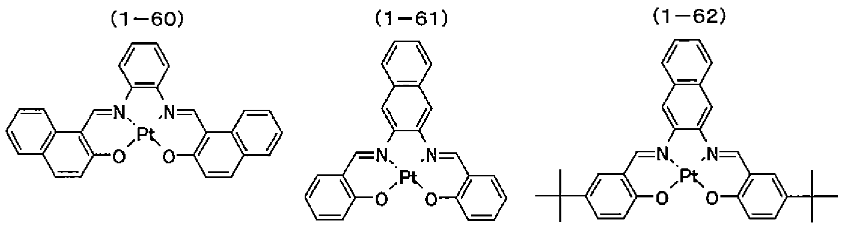

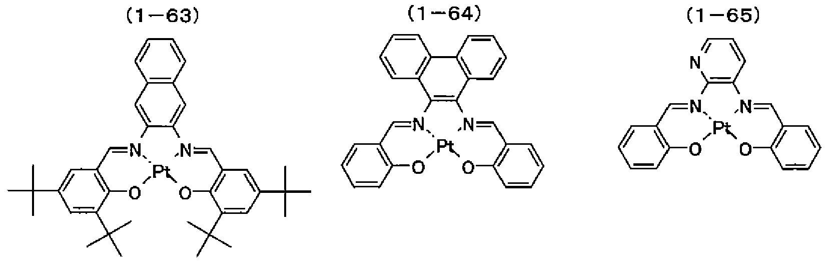

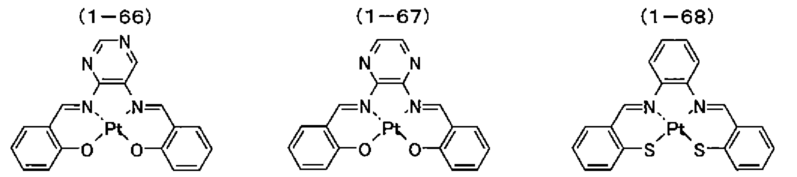

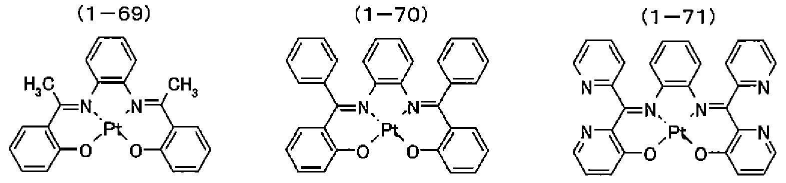

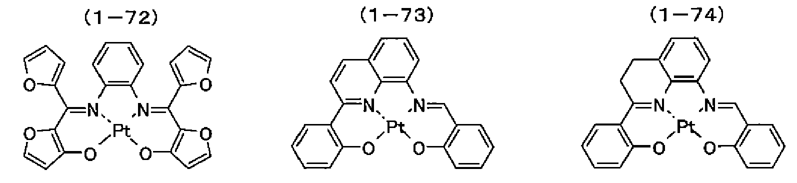

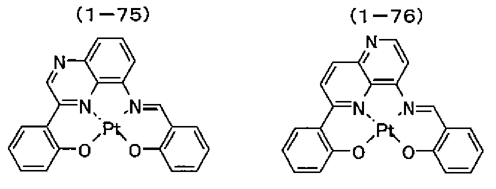

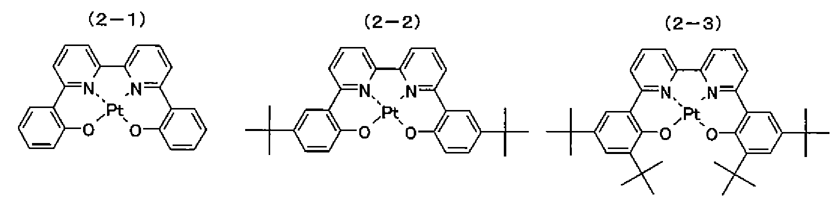

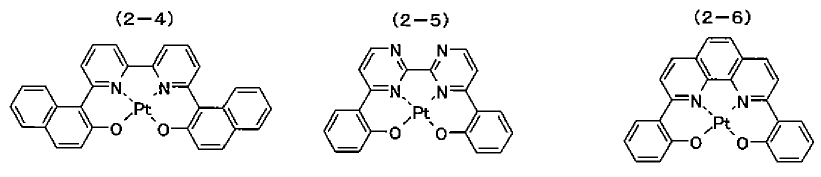

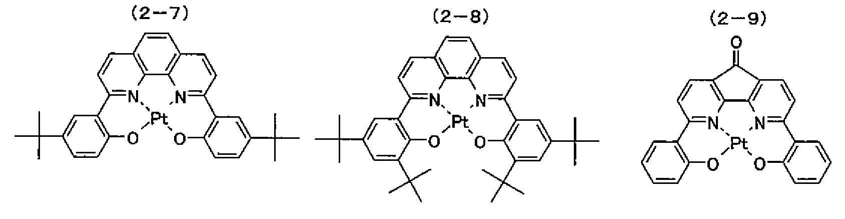

Hereinafter, methods of preparing the platinum complex represented by the general formula (1) (hereinafter, referred to as platinum complex (1)) and the platinum complex represented by the general formula (2) (hereinafter, referred to as platinum complex (2)) according to the present invention will be described with reference to the following reaction schemes.

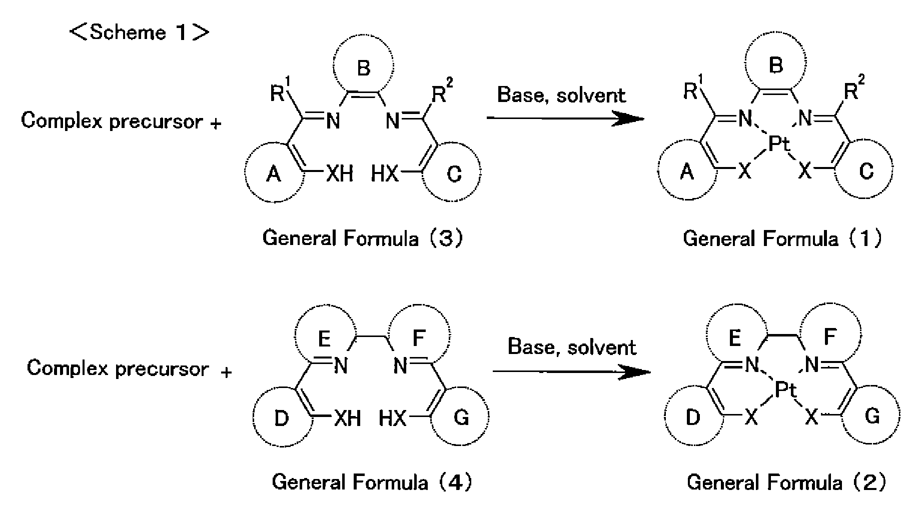

The platinum complex (1) can be produced easily by reacting a complex precursor with a compound represented by the general formula (3) (referred to as compound (3) hereinafter) in the presence of suitable base(s) and suitable solvent(s), under an inert gas atmosphere as needed.

Alternatively, the platinum complex (2) can be produced easily in a similar manner to the method of producing the platinum complex (1), by reacting a complex precursor with a compound represented by the general formula (4) (hereinafter, referred to as compound (4)) in the presence of suitable base (s) and suitable solvent(s), under an inert gas atmosphere as needed.

Hereinafter, raw compounds for the platinum complex of the present invention will be described.

In Scheme 1, the complex precursor may be an inorganic platinum compound or an organic platinum complex, and an organic platinum complex is more preferable. Favorable examples of the inorganic platinum compound include compounds represented by PtY₂ (Y represents a halogen atom.) and M₂PtY₄ (Y represents a halogen atom, and M represents an alkali metal.). The halogen atom represented by Y includes a fluorine atom, a chlorine atom, abromine atom, and iodine atom. The alkali metal represented by M includes lithium, sodium, potassium, and the like. Typical examples of the inorganic platinum compound include platinum (II) chloride, platinum (II) bromide, platinum (II) iodide, sodium chloroplatinate (II), potassium chloroplatinate (II), potassium bromoplatinate (II), and the like. Preferable organic platinum complexes are the platinum diene complexes represented by general formula (5):

Pt(H)Y₂

wherein, H represents a non-conjugated diene compound, and Y represents a halogen atom.

In the general formula(5),the non-conjugateddiene compound represented by H may be a cyclic or non-cyclic compound, and if the non-conj ugated diene compound is a cyclic non-conj ugated diene compound, it may be either mono-cyclic, poly-cyclic, fused cyclic, or bicyclo compound. The non-conjugated diene compound may be substituted with substituent group (s) . The substituents thereof are not particularly limited if they do not affect the production method of the present invention, and the substituents are preferably similar to the substituents described in detail in the description for the platinum complexes. Preferable non-conjugated diene compounds include 1,5-cyclooctadine, bicyclo [2, 2,1] hepta-2, 5-diene, 1,5-hexadiene, and the like. The non-conjugated diene compound is particularly preferably 1,5-hexadiene. Examples of the halogen atom represented by Y include a fluorine atom, a chlorine atom, a bromine atom, and iodine atom, and in particular, a chlorine atom and a bromine atom are preferable.

In Scheme 1, compounds (3) and (4) are tetradentate ligands having two nitrogen atoms capable of coordinating to platinum and two hydroxyl or mercapto groups capable of binding to the platinum. In the general formulae (3) and (4), rings A, B, C, D, E, F, and G, X, R¹ and R² are the same as those described above. Typical examples of the compounds (3) and (4) include compounds (3-1) to (3-76) and (4-1) to (4-9), which are compounds wherein in the compounds (1-1) to (1-76) and (2-1) to (2-9) represented as the typical examples of the platinum complexes of the invention, the platinum thereof were taken away and a hydrogen atom was bonded to each of atoms corresponding to the groups X in the general formulae (1) and (2).

The method for producing the platinum complex of the invention will be described hereinafter. Both the compounds (3) and (4) are called tetradentate ligands below for convenience. The amount of the tetradentate ligand used is normally 0.5 to 20 equivalents, preferably 0.8 to 10 equivalents, and more preferably 1.0 to 2.0 equivalents, with respect to the amount of complex precursor.

The production method of the present invention is preferably carried out in the presence of solvent(s). Preferable examples of the solvent include amides such as N,N-dimethylformamide, formamide, and N,N-dimethylacetamide; cyano-containing organic compounds such as acetonitrile; halogenated hydrocarbons such as dichloromethane, 1,2-dichloroethane, chloroform, carbon tetrachloride, and o-dichlorobenzene; aliphatic hydrocarbons such as pentane, hexane, heptane, octane, decane, and cyclohexane; aromatic hydrocarbons such as benzene, toluene, and xylene; ethers such as diethyl ether, diisopropyl ether, tert-butylmethyl ether, dimethoxy ethane, ethylene glycol diethyl ether, tetrahydrofuran, 1,4-dioxane, and 1,3-dioxolane; ketones such as acetone, methylethylketone, methylisobutylketone, and cyclohexanone; alcohols such as methanol, ethanol, 2-propanol, n-butanol, and 2-ethoxyethanol; polyvalent alcohols such as ethylene glycol, propylene glycol, 1,2-propanediol, and glycerol; esters such as methyl acetate, ethyl acetate, n-butyl acetate, and methyl propionate; sulfoxides such as dimethylsulfoxide; water, and the like. These solvents may be used alone or in combination of two or more as needed. More preferable examples of the solvent include amides such as N,N-dimethylformamide and N,N-dimethylacetamide; cyano-containing organic compounds such as acetonitrile; ethers such as ethylene glycol diethyl ether, tetrahydrofuran, 1,4-dioxane, and 1,3-dioxolane; ketones such as acetone, methylethylketone, and methylisobutylketone; alcohols such as methanol, ethanol, 2-propanol, n-butanol, and 2-ethoxyethanol; polyvalent alcohols such as ethylene glycol, propylene glycol, 1,2-propanediol, and glycerol; esters such as methylacetate, ethyl acetate, n-butyl acetate, and methyl propionate; water, and the like. These solvents may also be used alone or in combination of two or more as needed, and a solvent mixed with water is particularly preferable.

The amount of the solvent used is not particularly limited if the reaction proceeds smoothly, and the volume of the solvent is properly selected normally in the range by 1 to 200 parts, preferably by 1 to 50 parts, with respect to 1 part of complex precursor.

The production method of the present invention is favorably carried out in the presence of base (s) . The base is, for example, an inorganic or organic base. Favorable examples of the inorganic base include alkali metal hydroxides such as lithium hydroxide, sodium hydroxide, and potassium hydroxide; alkali metal carbonate salts such as lithium carbonate, sodium carbonate, and potassium carbonate; alkali metal bicarbonate salts such as sodium bicarbonate and potassium bicarbonate; metal hydrides such as sodium hydride; and the like. Preferable examples of the organic base include alkali metal alkoxides such as lithium methoxide, sodiummethoxide, potassiummethoxide, sodiumethoxide,potassium methoxide, and potassium tert-butoxide; organic amines such as triethylamine, diisopropylethylamine, N,N-dimethylaniline, piperidine, pyridine, 4-dimethylaminopyridine, 1,5-diazabicyclo[4,3,0]non-5-ene, l,8-diazabicyclo[5,4,0]undec-7-ene, tri-n-butylamine, and N-methylmorpholine; organic alkali metal compounds such as n-butyllithium and tert-butyllithium; Grignard reagents such as butylmagnesium chloride, phenylmagnesium bromide, and methylmagnesium iodide; and the like. The amount of the base used is properly selected normally in the range of 1 to 10 equivalents, preferably 1.5 to 5 equivalents, and more preferably 2 to 3 equivalents, with respect to that of the tetradentate ligand.

In the production method, three raw materials, the complex precursor, the tetradentate ligand and the base, may be mixed simultaneously to initiate reaction, or the complex precursor may be added to a reaction mixture obtained by reacting previously the tetradentate ligand and the base. Alternatively, the reaction mixture obtained by reacting previously the tetradentate ligand and the base may be added to the complex precursor.

The production method described above is preferably carried out under an inert gas atmosphere. Examples of the inert gas include nitrogen gas, argon gas, and other gases. The production method may also be carried out in combination with an ultrasonic generator. The reaction temperature is selected properly, normally in the range of 25 to 300°C, preferably 60 to 200°C, and more preferably 80 to 150°C. The reaction time differs depending on the reaction conditions such as reaction temperature, a solvent used, and a base used. Generally, the reaction time is selected properly in the range of normally 10 minutes to 72 hours, preferably 30 minutes to 48 hours, and more preferably 1 to 12 hours.

The platinum complex obtained may be additionally post-treated, isolated and purified, as needed. The post-treatment methods include, for example, extraction of reaction product, filtration of precipitate, crystallization by addition of solvent (s), distillation of solvent (s), and the like, and these post-treatments may be carried out alone or in combination. The methods for isolation or purification include, for example, column chromatography, recrystallization, sublimation, and the like, and these methods may be used alone or in combination.

The platinum complexes (1) and (2) of the present invention are useful as phosphorescent materials in light-emitting devices, in particular in organic EL devices.

Examples of the light-emitting device using the platinum complexes (1) and (2) of the present invention will be described hereinafter. The light-emitting device is not particularly limited in system, driving method, or application as long as it is a device utilizing the platinum complex of the present invention. A device utilizing light emission from the platinum complex or utilizing the platinum complex as a charge-transporting material is preferable. A typical light-emitting device is an organic EL device. The light-emitting device containing the platinum complex of the present invention will do as long as it contains at least one of the platinum complexes above, and in an emitting device having a light-emitting layer or organic compound multiple layers including an light-emitting layer between a pair of electrodes, at least one of the platinum complexes above is contained in at least one of the layers. The light-emitting device will do as long as at least one of the platinum complexes is contained, and the platinum complex may be contained in suitable combination of two or more.

The method of forming the organic layer (organic compound layer) containing the platinum complex of the present invention in the light-emitting device is not particularly limited. Examples thereof include a resistance-heating evaporative deposition method, an electron beammethod, sputtering, a molecule deposition method, a coating method, an inkjet method, and the like, and the resistance-heating evaporative deposition method and the coating method are preferable from the points of properties of the organic compound layer obtained and production efficiency.

The light-emitting device containing the platinum complex of the present invention is a device having a light-emitting layer or multiple-layered organic compound thin films containing a light-emitting layer formed between a pair of electrodes, anode and cathode, and may contain, in addition to the light-emitting layer, a hole injecting layer, a hole transporting layer, an electron injecting layer, an electron transporting layer, a protecting layer, or the like; and each of these layers may have another function as well. Any one of known materials may be used for formation of each of these layers.

The anode, which supplies holes to the hole injecting layer, the hole transporting layer, the light-emitting layer, and the like, is made of a material such as metal, alloy, metal oxide, electrically conductive compound, or the mixture thereof, that has preferably a work function of 4eV or more. Typical examples thereof include conductive metal oxides such as tin oxide, zinc oxide, indium oxide, and indium tin oxide (hereinafter, referred to as "ITO") ; metals such as gold, silver, chromium, and nickel; mixtures or deposits of the metal and the conductivity metal oxide; inorganic conductive substances such as copper iodide and copper sulfide; organic conductive materials such as polyaniline, polythiophene, and polypyrrole; and deposits of the material and ITO, and preferable are conductivity metal oxides. Of these, ITO is particularly preferable from the points, for example, of productivity, conductivity, and transparency. The anode layer is selected properly depending on the material used, and the thickness is normally, preferably in the range of 10 nm to 5 µm, more preferably 50 nm to 1 µm, and still more preferably 100 to 500 nm.

The anode is normally formed on a substrate, for example, of soda lime glass, non-alkali glass, transparent resin, or the like as a layer. When glass is used as a substrate material, use of a non-alkali glass is preferable from the viewpoint of reducing the amount of ions eluting from the glass. Alternatively, when soda lime glass is used as a substrate material, use of a glass barrier-coated, for example, with silica and the like is preferable. The thickness of the substrate is not particularly limited as long as the substrate has a sufficiently high mechanical strength, and if glass is used as a substrate material, the thickness of the glass substrate is normally 0.2 mm or more, preferably 0.7 mm or more. Various methods may be used for production of the anode depending on the material used, and, for example in production of an ITO anode, the ITO film is formed, for example, by an electron beammethod, a sputtering method, a resistance-heating evaporative deposition method, a chemical reaction method (e.g., sol-gel method), coating with an ITO dispersion, or the like. The drive voltage of the device can be reduced and the power efficiency of the device can be heightened, for example, by cleaning or other treatments of the anode. In the case of an ITO anode, for example, a UV-ozone or plasma treatment is effective.

The cathode injects electrons to the electron injecting layer, the electron transporting layer, the light-emitting layer, and the like, and is selected, taking into consideration the adhesiveness of the negative electrode to the neighboring layer such as electron injecting layer, the electron transporting layer, the light-emitting layer, or the like, as well as the ionization potential, stability, and others thereof. Examples of the cathode materials include metals, alloys, metal halides, metal oxides, electrically conductive compounds, and the mixtures thereof. Typical examples thereof include alkali metals such as lithium, sodium, and potassium and the fluorides thereof; alkali-earth metals such as magnesium and calcium and the fluorides thereof; gold, silver, lead, aluminum, sodium-potassiumalloy, and the mixed metalsthereof;magnesium-silver alloy or the mixed metals thereof; rare-earth metals such as indium and ytterbium; and the like. Favorable cathode materials have a work function of 4eV or less, more preferably aluminum, lithium-aluminum alloy or the mixed metals thereof, magnesium-silver alloy or the mixed metals thereof, and the like.

The cathode may have a deposited structure containing the compounds and the mixtures above. The thickness of the cathode layer is selected properly depending on the material used, and is selected normally, preferably in the range of 10 nm to 5 µm, more preferably 50 nm to 1 µm, and still more preferably 100 nm to 1 µm. Methods such as an electron beam method, a sputtering method, a resistance-heating evaporative deposition method, a coating method, or the like are used for production of the cathode, and in deposition, a single metal may be deposited or two or more metals may be deposited simultaneously. Alternatively, the cathode may be formed by simultaneous deposition of multiple metals or by deposition of an alloy previously prepared. And it is preferable that the sheet resistance of the cathode or the anode is lower.

The material for the light-emitting layer is not particularly limited as long as it provides a light-emitting layer with functions allowing injection of electrons from the anode or the hole injecting or hole transporting layer, and allowing emission of light by providing the field for recombination of the electrons with holes when an electric field is applied. The light-emitting layer may be doped with a fluorescent or phosphorescent material higher in power efficiency. Examples thereof include benzoxazole derivatives, triphenylamine derivatives, benzimidazole derivatives, benzothiazole derivatives, styrylbenzene derivatives, polyphenyl derivatives, diphenylbutadiene derivatives, tetraphenylbutadiene derivatives, naphthalimide derivatives, coumarin derivatives, perylene derivatives, perynone derivatives, oxadiazole derivatives, aldazine derivatives, pyrralizine derivatives, cyclopentadiene derivatives, bisstyrylanthracene derivatives, quinacridone derivatives, pyrrolopyridine derivatives, thiadiazopyridine derivatives, styrylamine derivatives, aromatic dimethylidyne compounds, various metal complex typically represented by metal or rare-earth complexes of 8-quinolinol derivatives, polymer compounds such as polythiophene, polyphenylene, and polyphenylenevinylene, organic silane derivatives, the platinum complexes of the present invention, and the like. The light-emitting layer may have a single layer structure consisting of one or more of the materials described above, or a multilayer structure having multiple layers of same composition or different compositions. The thickness of the light-emitting layer is not particularly limited, and is selected normally, preferably in the range of 1 nm to 5 *µ*m*,* more preferably 5 nm to 1 µm, and still more preferably 10 to 500 nm. The method of producing the light-emitting layer is not particularly limited, and examples thereof include methods such as an electron beam method, a sputtering method, a resistance-heating evaporative deposition method, a molecular depositing method, a coating method (e.g., spin coating, casting, dip coating, etc.), an inkjet method, an LB method, and the like; and preferable are the resistance-heating evaporative deposition method and coating methods.

The material for the hole injecting layer or the hole transporting layer should have a function of injecting holes from the anode, transporting the holes, or blocking electrons injected from the cathode. Typical examples of the materials for the hole injecting layer or the hole transporting layer include carbazole derivatives, triazole derivatives, oxadiazole derivatives, oxazole derivatives, imidazole derivatives, polyarylalkane derivatives, pyrazoline derivatives, pyrazolone derivatives, phenylenediamine derivatives, arylamine derivatives, amino-substituted chalcone derivatives, styrylanthracene derivatives, fluorenone derivatives, hydrazone derivatives, stilbene derivatives, silazane derivatives, aromatic tertiary amine compounds, styrylamine compounds, aromatic dimethylidyne compounds, porphyrin compounds, polysilane compounds, poly(N-vinylcarbazole) derivatives, aniline copolymers, thiophene oligomers, and conductive oligomers of polymers such as polythiophene, organic silane derivatives, the platinum complexes of the present invention, and the like. The thickness of the hole injecting layer or the hole transporting layer is not particularly limited, and is selected normally preferably in the range of 1 nm to 5 µm, more preferably 5 nm to 1 µm, and still more preferably 10 to 500 nm. The hole injecting layer or the hole transporting layer may have a single layer structure of one or more of the materials described above, or a multilayer structure consisting of multiple layers of same composition or different compositions. For production of the hole injecting layer or the hole transporting layer, methods such as a vacuum depositionmethod, an LB method, a method of coating a solution or dispersion of the hole injecting or the hole transporting agent in solvent (s) (e.g. , spin coating, casting, dip coating, etc.), an inkjet method, or the like is used. In the case of the coating methods, the materials above can be dissolved together with resin component(s) in solvent(s). Examples of the resin component include polyvinyl chloride, polycarbonate, polystyrene, polymethyl methacrylate, polybutyl methacrylate, polyester, polysulfone, polyphenylene oxide, polybutadiene, poly(N-vinylcarbazole), hydrocarbon resins, ketone resins, phenoxy resins, polyamide, ethylcellulose, polyvinyl acetate, ABS resins, alkyd resins, epoxy resins, silicone resins, and the like.

Any material may be used in the electron injecting or electron transporting layer, as long as it has a function of injecting electrons from the cathode, transporting the electrons, orblocking holes injected from the anode. The ionization potential of the hole blocking layer having a function of blocking holes injected from the anode is set to a value higher than that of the light-emitting layer. Typical examples of the material for the electron injecting or electron transporting layer include triazole derivatives, oxazole derivatives, polycyclic compounds, hetero polycyclic compounds such as bathocuproin, oxadiazole derivatives, fluorenone derivatives, diphenylquinone derivatives, thiopyran dioxide derivatives, anthraquinonedimethane derivatives, anthrone derivatives, carbodiimide derivatives, fluorenylidenemethane derivatives,distyrylpyrazine derivatives, acid anhydrides for example of naphthalenetetracarboxylic acid and perylenetetracarboxylic acid, phthalocyanine derivatives, various metal complexes of the compounds represented by metal complexes of 8-quinolinol derivatives, metal phthalocyanines, and metal complexes having benzoxazole or benzothiazole as a ligand, organic silane derivatives, the platinum complexes according to the present invention, and the like. The thickness of the electron injecting or electron transporting layer is not particularly limited, and is selected normally, preferably in the range of 1 nm to 5 µm, more preferably 5 nm to 1 µm, and still more preferably 10 to 500 nm. The electron injecting or electron transporting layer may have a single layer structure consisting of one or more of the materials described above material or a multilayer structure having multiple layers consisting of same composition or different compositions. For production of the electron injecting or electron transporting layer, methods such as a vacuum deposition method, an LB method, a method of coating a solution or dispersion of the electron injecting or electron transporting agent above in a solvent (e.g., spin coating, casting, dip coating, etc.), an inkjet method, or the like may be used. In the case of the coating methods, the materials can be dissolved or dispersed together with resins component in solvent (s), and the compounds exemplified for the hole injecting layer and hole transporting layer may be used as the resin components.

Any material may be used as the material for protecting layer as long as it has a function of preventing intrusion of the compounds that accelerate degradation of the device such as water and oxygen into the device. Typical examples of the material for protecting layer include metals such as indium, tin, lead, gold, silver, copper, aluminum, titanium, and nickel; metal oxides such as magnesium oxide, silicon dioxide, dialuminum trioxide, germanium oxide, nickel oxide, calcium oxide, barium oxide, diiron trioxide, diytterbium trioxide, and titanium oxide; metal fluorides such as magnesium fluoride, lithium fluoride, aluminum fluoride, and calcium fluoride; polyethylene, polypropylene, polymethyl methacrylate, polyimide, polyurea, polytetrafluoroethylene, polychlorotrifluoroethylene, polydichlorodifluoroethylene, copolymers of chlorotrifluoroethylene and dichlorodifluoroethylene, copolymers obtained by copolymerizing a monomer mixture containing tetrafluoroethylene and at least one comonomer, fluorine-containing copolymers having a cyclic structure in the copolymer main chain, water-absorbing substances having a water absorption of 1% or more, moisture-proof substances having a water absorption of 0 .1% or less, and the like. The method of producing the protecting layer is also not particularly limited, and can be prepared, for example, by a vacuum deposition method, a sputtering method, a reactive sputtering method, an MBE (molecular beam epitaxy) method, a cluster ion beam irradiation method, an ion plating method, a plasma polymerization (high-frequency wave excitation ion plating) method, a plasma CVD method, a laser CVD method, a thermal CVD method, a gas source CVD method, or a coating method.

### BEST MODE FOR CARRYING OUT THE INVENTION

Hereinafter, the present invention will be described more specifically with reference to Examples, Reference Examples, Application Examples and Comparative Application Examples, but it should be understood that the present invention is not limited by these examples at all.

The apparatuses used for measurement in Examples and Reference Examples are as follows:
(1) 200 MHz ¹H-NMR spectrum analysis: GEMINI 2000 (manufactured by Varian)
(2) 500 MHz ¹H-NMR spectrum analysis: DRX-500 (manufactured by Bruker)
(3) UV-Visible Spectrometer: V-550 (manufactured by JASCO)
(4) Fluorescence Spectrometer: F-4500 (manufactured by Hitachi)

### Reference Example 1

### Preparation of exemplary compound (3-3): 2,2'-[1,2-phenylene-bis(nitrilomethylidyne)]bis(4-tert-butylphenol)

To an ethanol (5 mL) solution of 1,2-phenylenediamine (276 mg, 2.55 mmol, 1.0 equivalent), an ethanol (5 mL) solution of 5-tert-butylsalicylaldehyde (1.00 g, 5.61 mmol, 2.2 equivalents) was added dropwise, and the mixture was stirred under reflux for 8 hours. Crystals in the orange-colored suspension obtained were filtered, washed with ethanol and hexane, and dried by heating under reduced pressure, to give 670 mg of the exemplary compound (3-3) as orange-colored powder. Yield: 61.3%.
¹H NMR (200 MHz, CDCl₃): 1.32 (s, 18H), 6.99 (d, J=8.6Hz, 2H), 7.15-7.48 (m, 8H), 8.64 (s, 2H), and 12.84 (s, 2H).

### Reference Example 2

### Preparation of exemplary compound (3-4): 2,2'-[1,2-phenylene-bis(nitrilomethylidyne)]bis(4,6-di-tert-butylphenol)

To a mixture of 1,2-phenylenediamine (1.47 g, 13.58 mmol, 1.0 equivalent) and 3,5-di-tert-butylsalicylaldehyde (7.00 g, 29.87 mmol, 2.2 equivalents), ethanol (100 mL) was added, and then the mixture was stirred under reflux for 40 hours. Crystals were filtered from the yellow orange-colored suspension obtained and dried by heating under reduced pressure, to give 5.90 g of the exemplary compound (3-4) in a form of yellow powder. The filtrate was concentrated to 20 mL and then stirred additionally under reflux for 48 hours to obtain yellow orange-colored suspension. Crystals were filtered from the yellow orange-colored suspension obtained and dried by heating under reduced pressure, to give still more 1.20 g of the exemplary compound (3-4) as yellow powder. Total yield: 96.6%.
¹H NMR (500 MHz, CD₂Cl₂) : 1.32 (s, 18H), 1.42 (s, 18H), 7.26 (d, J=2.4Hz, 2H), 7.27-7.37 (m, 4H), 7.45 (d, J=2.4Hz, 2H), 8.69 (s, 2H), and 13.59 (s, 2H).

### Reference Example 3

### Preparation of exemplary compound (3-15): 2,2'-[1,2-phenylene-bis(nitrilomethylidyne)]bis(4-methoxy-phenol)

To an ethanol (20 mL) solution of 1, 2-phenylenediamine (787 mg, 7.28 mmol, 1.0 equivalent), added dropwise was 5-methoxysalicylaldehyde (2. 0 mL, 16. 02 mmol, 2.2 equivalents), and then the mixture was stirred at room temperature for 8 hours. Crystals were filtered from the orange-colored suspension obtained and dried by heating under reduced pressure, to give 3.4 g of the exemplary compound (3-15) in a form of red orange-colored powder. Yield: 96.5%.
¹H NMR (200 MHz, CDCl₃) : 3. 80 (s, 6H), 6.89 (t, J=1.8Hz, 2H), 6.99 (d, J=1.8Hz, 4H), 7.20-7.40 (m, 6H), 8.60 (s, 2H), and 12.58 (s, 2H) .

### Reference Example 4

### Preparation of exemplary compound (3-16): 2,2'-[1,2-phenylene-bis(nitrilomethylidyne)]bis(6-methoxyphenol)

To a mixture of 1,2-phenylenediamine (1.0 g, 9.25 mmol, 1. 0 equivalent) and o-vanillin (3.1g, 20.35 mmol, 2.2 equivalents), added was ethanol (100 mL), and then the mixture was stirred at room temperature for 8 hours. Crystals were filtered from the orange-colored suspension obtained and dried by heating under reduced pressure, to give 3.2 g of the exemplary compound (3-16) in a form of red orange-colored powder. Yield: 91.9%.
¹H NMR (200 MHz, CDCl₃): 3.90 (s, 6H), 6.86 (dd, J=7.6, 8.2Hz, 2H), 6.99 (t, J=7.4Hz, 2H), 7.00 (t, J=7.4Hz, 2H), 7.15-7.40 (m, 4H), 8.63 (s, 2H), and 13.17 (s, 2H).

### Reference Example 5

### Preparation of exemplary compound (3-61): 2,2'-[2,3-naphthalendiyl-bis(nitrilomethylidyne)]bisphenol

To an ethanol (150 mL) solution of 2,3-diaminonaphthalene (1.0 g, 6.32 mmol, 1.0 equivalent), added dropwise was salicylaldehyde (1.5 mL, 13. 90 mmol, 2.2 equivalents), and then the mixture was stirred under reflux for 8 hours. Crystals were filtered from the orange-colored suspension obtained and dried by heating under reduced pressure, to give 1.9 g of the exemplary compound (3-61) in a form of orange-colored powder. Yield: 81.3%. ¹H NMR (200 MHz, CDCl₃): 6.95 (dt, J=1.0, 7.2Hz, 2H), 7.07 (d, J=8.0Hz, 2H), 7.34-7.55 (m, 6H), 7.60 (s, 2H), 7.81-7.95 (m, 2H), 8.75 (s, 2H), and 13.02 (s, 2H).

### Reference Example 6

### Preparation of exemplary compound (3-62): 2,2'-[2,3-naphthalendiyl-bis(nitrilomethylidyne)]bis(4-tert-butylphenol)

To an ethanol (100 mL) solution of 2,3-diaminonaphthalene (807 mg, 5.10 mmol, 1.0 equivalent), added dropwise was 5-tert-butylsalicylaldehyde (2.0g, 11.22mmol, 2.2 equivalents), and then the mixture was stirred under reflux for 15 hours. Crystals were filtered from the orange-colored suspension obtained and dried by heating under reduced pressure, to give 1.44 g of the exemplary compound (3-62) in a form of orange-colored powder. Yield: 59.0%.
¹H NMR (200 MHz, CDCl₃): 1.33 (s, 18H), 7.01 (dd, J=1.0, 8.2Hz, 2H), 7.41 (s, 2H), 7.45-7.55 (m, 4H), 7.59 (s, 2H), 7.80-7.92 (m, 2H), 8.75 (s, 2H), and 12.81 (s, 2H).

### Reference Examples 7-1 to 7-3

### Preparation of exemplary compound (4-1): 2,2'-(2,2'-bipyridine)-6,6'-diyl-bisphenol

### Reference Example 7-1

### Preparation of 6-(2-methoxyphenyl)-2,2'-bipyridine

A reaction flask equipped with a reflux condenser and a dropping funnel was dried by heating under reduced pressure, and the internal gas was substituted with nitrogen. Then, diethylether (20 mL) and lithium metal cubes (702 mg, 101. 18 mmol, 2.1 equivalents with respect to 2-bromoanisole) were placed in a reactor, and a diethylether (20 mL) solution of 2-bromoanisole (6.0 mL, 48.18 mmol, 1.5 equivalents) was added dropwise thereto over a period of 1 hour at a dropping velocity adjusted to make the content reflux gently. After dropwise addition, the mixture was heated under reflux additionally for 1 hour, to give a diethyl ether solution of 2-methoxyphenyllithium.

2,2'-bipyridine (5.0 g, 32.01 mmol, 1.0 equivalent) was placed in a reactor, and the internal gas was substituted with nitrogen. Then, after addition of diethyl ether (100 mL), the mixture was cooled to 5°C in an ice bath. The diethyl ether solution of 2-methoxyphenyllithium prepared above was added dropwise to the mixture over a period of 1 hour, and the mixture was stirred at room temperature additionally for 12 hours. The black purple reaction solution obtained was poured into a saturated aqueous ammonium chloride solution slowly, and after separation of the organic layer, the aqueous layer was extracted with methylene chloride. The organic layers were combined and concentrated under reduced pressure; the residue obtained was dissolved in acetone (50 mL) ; and an acetone (200 mL) solution of potassium permanganate (2.0 g, 12.66 mmol, 0.4 equivalents) was added thereto dropwise. Celite was added to the brown suspension obtained after dropwise addition; the mixture was suction-filtered; and the filtrate was concentrated, roughly purified by silica gel column chromatography (eluent: dichloromethane/ethyl acetate: 15/1), to give 3.9 g of an intermediate 6-(2-methoxyphenyl)-2,2'-bipyridine in a form of red viscous oil. Yield: 46.4%. The intermediate was used in the next reaction without further purification.
¹H NMR (200 MHz, CaCl₃) : 3.88 (s, 3H), 7.03 (d, J=8.2Hz, 1H), 7.13 (dt, J=1.0, 7.4Hz, 1H), 7.29 (ddd, J=1.2, 4.8, 7.6Hz, 1H), 7.40 (ddd, J=1.0, 1.8, 7.4Hz, 1H), 7.74-7.84 (m, 2H), 7.92 (dd, J=1.2, 7.8Hz, 1H), 8.01 (dd, J=1.8, 7.8Hz, 1H), 8.34 (dd, J=1.2, 7.6Hz, 1H), 8.57 (dt, J=8.0, 1.0Hz, 1H), and 8. 68 (ddd, J=1.0, 1.8, 4.8Hz, 1H).

### Reference Example 7-2

### Preparation of 6,6'-di(2-methoxyphenyl)-2,2'-bipyridine

A diethylether solution of 2-methoxyphenyllithium was prepared in a similar manner to above from lithium metal cubes (325 mg, 46.85 mmol, 2.1 equivalents with respect to 2-bromoanisole), 2-bromoanisole (2.8 mL, 22.31 mmol, 1.5 equivalents) and diethylether (20 mL).

A diethylether (80 mL) solution of 6-(2-methoxyphenyl)-2,2'-bipyridine prepared in Reference Example 7-1 (3.9 g, 14.87 mmol, 1.0 equivalent) was placed in a nitrogen-substituted reaction flask and cooled to 5°C in an ice bath. To the solution, the diethyl ether solution of 2-methoxyphenyllithium prepared above was added over a period of 1 hour, and after dropwise addition, the mixture was stirred at room temperature additionally for 12 hours. The black purple reaction solution obtained was added to a saturated aqueous ammonium chloride solution slowly, and after separation of the organic layer, the aqueous layer was extracted with methylene chloride. The organic layers were combined and concentrated under reduced pressure; the residue obtained was dissolved in acetone (50 mL) ; and an acetone (100 mL) solution of potassiumpermanganate (1.0 g, 6.33 mmol, 0.4 equivalents) was added dropwise thereto. Celite was added to the suspension obtained after dropwise addition; the mixture was suction-filtered; and the filtrate was concentrated, then purified by silica gel column chromatography (eluent:dichloromethane), and recrystallized from hexane, to give 2.5 g of an intermediate, 6,6'-di(2-methoxyphenyl)-2,2'-bipyridine, in a form of cream-colored powder. Yield: 45.6%.
¹H NMR (200 MHz, CDCl₃) : 3. 90 (s, 6H), 7.04 (d, J=8 .4Hz, 2H), 7.14 (dt, J=1.0, 7.6Hz, 2H), 7.40 (ddd, J=1.0, 1.8, 7.4Hz, 2H), 7.82 (t, J=7.4Hz, 2H), 7.92 (dd, J=1.4, 8.0Hz, 2H), 8.04 (dd, J=1.8Hz, 7.6Hz, 2H), and 8.50 (dd, J=1.4, 7.4Hz, 2H).

### Reference Example 7-3

### Preparation of exemplary compound (4-1): 2,2'-(2,2'-bipyridine)-6,6'-diylbisphenol)

Pyridine (10. 5 mL, 130.20 mmol, 20. 0 equivalents) was placed in a reaction flask, and concentrated hydrochloric acid (15. 6 mL, 130.20 mmol, 20.0 equivalents) was added dropwise thereto. The solution obtained was heateduntil the internal temperature reaches 200°C, with removal of water. The content was cooled to 140°C; 6,6'-di(2-methoxyphenyl)-2,2'-bipyridine prepared in Reference Example 7-2 (2.4 g, 6.51 mmol, 1.0 equivalent) was added thereto; and the mixture was stirred at 200°C for 3 hours. Yellow solid obtained by cooling the content was added into a suspension of dichloromethane and water, and 100 mL of an aqueous 1N sodium hydroxide solution was added dropwise thereto until the pH of the aqueous layer became 7.0. After separation of the organic layer, the aqueous layer was extracted with dichloromethane three times, and the organic layers were combined and concentrated under reduced pressure. The residue obtained was purified by silica gel column chromatography (eluent: dichloromethane) and recrystallized from hexane/dichloromethane, to give 2.0 g of the exemplary compound (4-1) in a form of cream-colored powder. Yield: 90.3%.
¹H NMR (500 MHz, CD₂Cl₂) : 6.97 (ddd, J=1.2, 7. 1, 8.0Hz, 2H), 7.03 (dd, J=1.2, 8.3Hz, 2H), 7.35 (ddd, J=1.6, 7.1, 8.3Hz, 2H), 7.91 (dd, J=1.6, 8.0Hz, 2H), 8.05 (dd, J=1.7, 7.9Hz, 2H), 8.08 (dd, J=7.4, 7.9Hz, 2H), 8.11 (dd, J=1.7, 7.4Hz, 2H), and 14.10 (s, 2H).

### Example 1

### Preparation of exemplary compound (1-3): [[2,2'-[1,2-phenylene-bis(nitrilomethylidyne)]bis[4-tert-butylphenolate]]-N,N',O,O']platinum (II)

Potassium chloroplatinate (II) (500 mg, 1.20 mmol, 1.0 equivalent), the exemplary compound (3-3) prepared in Reference Example 1 (570 mg, 1.32 mmol, 1.1 equivalents), and potassium hydroxide (168 mg, 3.00 mmol, 2.5 equivalents) were placed in a Schlenk flask and the internal gas was substituted with nitrogen. Then, acetone (10 mL) and water (10 mL) were added thereto in that order, and the mixture was stirred under reflux for 1 hour. After recovery of acetone from the suspension obtained, the crude crystals precipitated were filtered and washed with a saturated aqueous sodium bicarbonate solution. The crude crystals were purified by silica gel column chromatography (eluent: dichloromethane/ethyl acetate: 20/1), to give 321 mg of the exemplary compound (1-3) in a form of purple powder. Yield: 43.0%. For use in production of an organic EL device, the compound is further purified by sublimation (1.3x10⁻⁵ Torr, 330°C) in a sublimation yield of 81.4%, to give the exemplary compound (1-3) in a form of red powder.
¹H NMR (500 MHz, CD₂Cl₂): 1.35 (s, 18H), 7.18 (d, J=9.1Hz, 2H), 7. 33 (dt, J=9. 6, 3. 3Hz, 2H), 7. 50 (d, J=2.6Hz, 2H), 7.66 (dd, J=2.6, 9.1Hz, 2H), 7.98 (dt, J=9.6, 3.3Hz, 2H), and 8.87 (s, 2H).

### Example 2

### Preparation of exemplary compound (1-4): [[2,2'-[1,2-phenylene-bis(nitrilomethylidyne)]bis[4,6-di-tert-butylphenolate]]-N,N',O,O]platinum (II)

[(1,2,5,6-η⁴)-1,5-hexadienyl]platinum(II) dichloride (300 mg, 0.862 mmol, 1.0 equivalent), the exemplary compound (3-4) prepared in Reference Example 2 (513 mg, 0.948 mmol, 1.1 equivalents) and potassium hydroxide (121 mg, 2.155 mmol, 2.5 equivalents) were placed in a Schlenk flask, and the internal gas was substituted with nitrogen. Then, 2-ethoxyethanol (30 mL) was added thereto, and the mixture was stirred under reflux for 1 hour. The residue obtained after evaporation of the solvent from the reaction solution was purified by silica gel column chromatography (eluent: hexane/toluene: 1/1) and recrystallization (solvent: methanol), to give 432 mg of the exemplary compound (1-4) in a form of red powder. Yield: 68.3%. For use in production of an organic EL device, the compound is further purified by sublimation (8.0 × 10⁻⁶ Torr, 300°C) in a sublimation yield of 82.1%, to give the exemplary compound (1-4) in a form of red powder.
¹H NMR (500 MHz, CD₂Cl₂) : 1.36 (s, 18H), 1.56 (s, 18H), 7.30-7.34 (m, 2H), 7.38 (d, J=2.6Hz, 2H), 7.68 (d, J=2.6Hz, 2H), 7.98-8.03 (m, 2H), and 8.91 (s, 2H).
Fluorescence wavelength: 639.6 nm (excitation wavelength: 575.0 nm)

### Example 3

### Preparation of exemplary compound (1-15): [[2,2'-[1,2-phenylene-bis (nitrilomethylidyne)]bis [4-methoxyphenolate]] - N,N',O,O']platinum (II)

Potassium chloroplatinate (II) (500 mg, 1.20 mmol, 1.0 equivalent), the exemplary compound (3-15) prepared in Reference Example 3 (499 mg, 1.32 mmol, 1.1 equivalents) and potassium hydroxide (168 mg, 3.00 mmol, 2.5 equivalents) were placed in a Schlenk flask, and the internal gas was substituted with nitrogen. Then, acetone (10 mL) and water (10 mL) were added thereto in that order, and the mixture was stirred under reflux for 1 hour. After recovery of acetone from the suspension obtained, the crude crystals precipitated were filtered and washed with a saturated aqueous sodium bicarbonate solution. The crude crystals were purified by silica gel column chromatography (eluent: dichloromethane/methanol: 20/1), to give 367 mg of the exemplary compound (1-15) in a form of black brown powder. Yield: 53.7%. ¹H NMR (500 MHz, CD₂Cl₂) : 3.82 (s, 6H), 6.98 (d, J=3.2Hz, 2H), 7.19 (d, J=9.4Hz, 2H), 7.28 (dd, J=3.2, 9.4Hz, 2H), 7.36 (dt, J=9.6, 3.3Hz, 2H), 8.00 (dt, J=9.7, 3.3Hz, 2H), and 8.86 (s, 2H).

### Example 4

### Preparation of exemplary compound (1-61): [[2,2'-[2,3-naphthalendiyl-bis(nitrilomethylidyne)]bis[phenolate]]-N,N',O,O']platinum (II)

Potassium chloroplatinate (II) (500 mg, 120 mmol, 1.0 equivalent), the exemplary compound (3-61) prepared in Reference Example 5 (487 mg, 1.32 mmol, 1.1 equivalents) and potassium hydroxide (168 mg, 3.00 mmol, 2.5 equivalents) were placed in a Schlenk flask and the internal gas was substituted with nitrogen. Then, acetone (10 mL) and water (10 mL) were added thereto in that order, and the mixture was stirred under reflux for 1 hour. After recovery of acetone from the suspension obtained, the crude crystals precipitated were filtered and washed with a saturated aqueous sodium bicarbonate solution. The crude crystal was purified by silica gel column chromatography (eluent: dichloromethane/ethyl acetate: 10/1 and then dichloromethane/methanol =20/1), to give 377 mg of an exemplary compound (1-61) in a form of dark purple powder. Yield: 56.2%. For use in production of an organic EL device, the compound is further purified by sublimation (8.0 × 10⁻⁶ torr, 340°C) in a sublimation yield of 80.0%, to give the exemplary compound (1-61) in a form of dark purple powder.
¹H NMR (500 MHz, CD₂Cl₂) : 6.81 (ddd, J=1.1, 6.7, 7.9Hz, 2H), 7.25 (d, J=8.6Hz, 2H), 7.56-7.64 (m, 4H), 7.69 (dd, J=1.8, 8.1Hz, 2H), 7.96-8.04 (m, 2H), 8.42 (s, 2H), and 9.13 (s, 2H).

### Example 5

### Preparation of exemplary compound (1-62): [[2,2'-[2,3-naphthalendiyl-bis(nitrilomethylidyne)]bis[4-tert-butylphenolate]]-N,N',O,O']platinum (II)

Potassium chloroplatinate (II) (500 mg, 1.20 mmol, 1.0 equivalent), the exemplary compound (3-62) prepared in Reference Example 6 (636 mg, 1.32 mmol, 1.1 equivalents) and potassium hydroxide (168 mg, 3.00 mmol, 2.5 equivalents) were placed in a Schlenk flask and the internal gas was substituted with nitrogen. Then, acetone (10 mL) and water (10 mL) were added thereto in that order, and the mixture was stirred under reflux for 1 hour. After recovery of acetone from the suspension obtained, the crude crystals precipitated were filtered and washed with a saturated aqueous sodium bicarbonate solution. The crude crystals were purified by silica gel column chromatography (eluent: dichloromethane/ethyl acetate =50/1), to give 333 mg of an exemplary compound (1-62) in a cotton-like state red substance. Yield: 41. 3%. For use in production of an organic EL device, the compound is further purified by sublimation (4 . 0 × 10⁻⁶ torr, 330°C) in a sublimation yield of 87.2%, to give the exemplary compound (1-62) in a form of black purple crystal.
¹H NMR (500 MHz, CD₂Cl₂): 1.37 (s, 18H), 7.16 (d, J=9.0Hz, 2H), 7.48-7.56 (m, 4H), 7.67 (dd, J=2.6, 9.0Hz, 2H), 7.92 (dt, J=9.5, 3.2Hz, 2H), 8.35 (s, 2H), 9.05 (s, 2H).

### Example 6

### Preparation of exemplary compound (2-1): [2,2'-(2,2'-bipyridine)-6,6'-diyl-bis[phenolate]]-N,N',O,O']platinum (II)

[(1,2,5,6-η⁴)-1,5-hexadienyl]platinum (II) dichloride (100 mg, 0.287mmol, 1.0 equivalent), the exemplary compound (4-1) prepared in Reference Example 7-3 (108 mg, 0.316 mmol, 1.1 equivalents) and potassium hydroxide (40 mg, 0.718 mmol, 2.5 equivalents) were placed in a Schlenk flask and the internal gas was substituted with nitrogen. Then, 2-ethoxyethanol (10 mL) was added thereto, and the mixture was stirred under reflux for 1 hour. Crystals were filtered from the brown suspension obtained and washed with hexane. The crude crystal was purified by silica gel column chromatography (eluent: methylene chloride/methanol: 20/1) and recrystallized from hexane/dichloromethane, to give 119 mg of the exemplary compound (2-1) in a form of red orange-colored powder. Yield: 77.7%.
¹H NMR (500 MHz, CD₂Cl₂) : 6.80 (ddd, J=1.1, 6.7, 8.1Hz, 2H), 7.29 (br d, J=8.5Hz, 2H), 7.39 (ddd, J=1.7, 6.7, 8.3Hz, 2H), 7.92 (br d, f=7.9Hz, 2H), 8.00 (br d, J=8.3Hz, 2H), 8.08-8.15 (m, 2H), and 8.20-8.25 (m, 2H).

### Application Example 1

An organic EL device having the layer structure shown in Fig. 1 was prepared by forming, on a glass substrate (g), an anode (f), a hole transporting layer (e), an light-emitting layer (d) consisting of a host material and a dope material, a hole blocking layer (c), an electron transporting layer (b) and a cathode (a) in that order from the glass substrate (g). In the organic EL device, the anode (f) and the cathode (a) have lead wires respectively connected, and a voltage can be applied between the anode (f) and the cathode (a) through the wires. Typical materials and production methods of each layer will be described briefly below.

First, the anode (f) is made of an ITO film, and deposited on a glass substrate (g) . The hole transporting layer (e) is formed to be 40 nm in thickness on the anode (f) by vacuum deposition of the compound (α-NPD) represented by the following Formula:

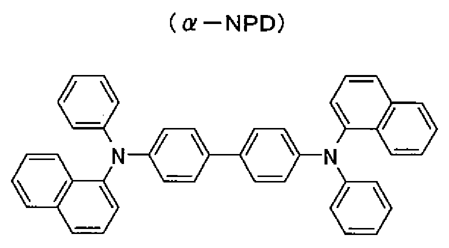

The light-emitting layer (d) containing a host material and a doped phosphorescence light-emitting material is formed to be 35 nm in thickness on the hole transporting layer (e) by simultaneous vacuum deposition (dope 3%) of the compound (CBP) represented by the following Formula: 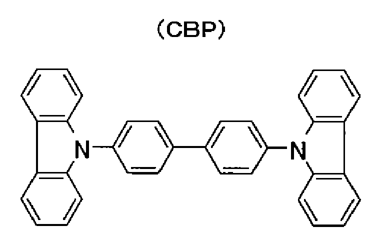 and the compound (1-4) prepared in Example 2 and represented by the following Formula:

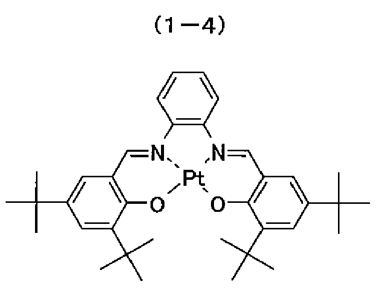

In addition, the hole blocking layer (c) is formed to be 10 nm in thickness on the light-emitting layer (d) by vacuum deposition of the compound (BCP) represented by the following Formula:

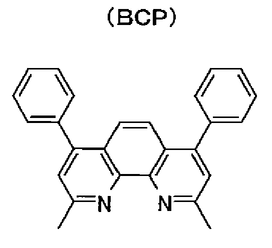

The electron transporting layer (b) is formed to be 35 nm in thickness on the hole blocking layer (c) by vacuum deposition of the compound (Alq₃) represented by the following Formula:

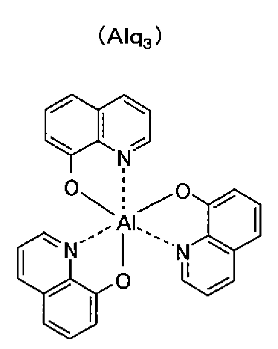

The cathode (a) is formed as a deposited film by vacuum co-deposition of Mg and Ag at a ratio of 10 : 1 to be 100 nm in thickness and then vacuum deposit ion of Ag additionally to be 10 nmin thickness in that order from the side of the electron transporting layer (b) .

When a plus voltage is applied to the anode (ITO) (f) of the organic EL device obtained and a minus voltage to the cathode (a), stabilized light emission was confirmed even at a very low voltage. The external quantum efficiency of the device showed extremely high value of 5.0% at a brightness of 100 cd/m². Also observed was emission of red light extremely high in color purity derived from the compound (1-4) used in the light-emitting layer (d) .

The characteristics of the organic EL device prepared in Application Example 1 are summarized in the following Table 1.

**Table 1: Characteristics of the device prepared**

| App. Ex. No. | Emitting layer | EL peak (nm) | CIE Chromaticity point at 100 cd/m² | External quantum efficiency at 100 cd/m² (%) | Power Efficiency at 100 cd/m² (1m/W) |
|---|---|---|---|---|---|
| 1 | 1-4(3%), CBP | 647.1 | 0.67, 0.30 | 5.0 | 1.0 |

### Comparative Application Example 1

A device having a structure similar to that of the device of Application Example 1 was prepared by using a known red phosphorescent material, (2,3,7,8,12,13,17,18-octaethyl-21H,23H-porphinato-N,N,N,N)platinum (II) [Pt(OEP)] that is superior in performance and represented by the following Formula: 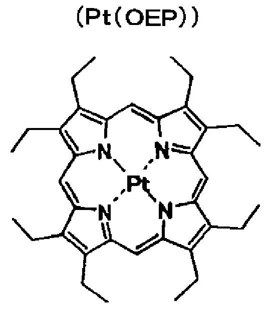 instead of the platinum complex (1-4) in the light-emitting layer (d). The characteristics of the organic EL device are summarized in Table 2.

**Table 2: Characteristics of the device prepared**

| Com. App. Ex. No. | Emitting layer | EL peak (nm) | CIE Chromaticity Point at 100 cd/m² | External quantum efficiency at 100 cd/m² (%) | Power Efficiency at 100 cd/m² (1m/W) |
|---|---|---|---|---|---|
| 1 | Pt (OEP) (3%), CBP | 648 | 0.72, 0.27 | 2.6 | 0.3 |

Comparison between the characteristics of the organic EL devices prepared in Application Example 1 and Comparative Application Example 1 reveals that both devices emit red light higher in color purity. On the other hand, as for the efficiency of device, the device of Application Example 1 has an external quantum efficiency higher by 1.92 times and a power efficiency higher by 3.33 times than those of the device of Comparative Application Example 1. As apparent from these results, the organic EL device containing the platinum complex of the invention is extremely superior in color purity, external quantum efficiency and power efficiency.

### ADVANTAGEOUS EFFECTS OF THE INVENTION

As described above in detail, the platinum complex of the present invention is extremely superior inheat stability, emission characteristics, and power efficiency, useful in particular as a phosphorescent material, and applicable to production of various display devices, especially high-efficiency organic EL devices.

### INDUSTRIAL APPLICABILITY

The platinum complex of the present invention can be used favorably as a light-emitting material for a display device, a display device, a back light unit, an electrophotographic machine, an illumination light source, a record light source, a light-exposure source, a read light source, and an emitting devices for signs and marks, signboards, interior goods, and the like.

## Claims

1. Platinum complex represented by general formula (1): 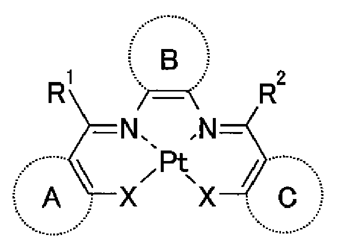
wherein, rings A, B and C each independently represent an aromatic ring optionally having substituent (s) or an aromatic heterocyclic ring optionally having substituent (s) ; X represents an oxygen atom or a sulfur atom; R¹ and R² each independently represent a hydrogen atom or a substituent; and more, R¹ and the ring B, and also R² and the ring B each may together form a fused ring;
with the proviso that excluding the following platinum complex:

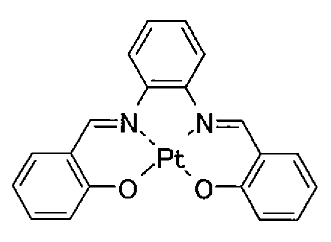

2. Platinum complex represented by general formula (2): 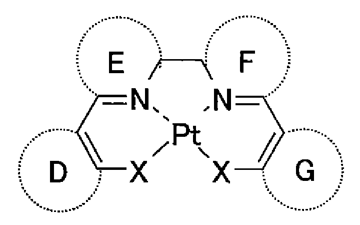
wherein, rings D and G each independently represent an aromatic ring optionally having substituent (s) or an aromatic heterocyclic ring optionally having substituent (s) ; X represents an oxygen atom or a sulfur atom; rings E and F each independently represent nitrogen-containing aromatic heterocyclic ring optionally having substituent (s) ; and more, rings E and F may together form a fused ring.
